# EUROPEAN PATENT SPECIFICATION

(11) **EP 2 566 554 B1**
(45) Date of publication and mention of the grant of the patent: **01.04.2015**
(21) Application number: 11716808.8
(22) Date of filing: 15.04.2011
(51) Int. Cl.: A61M 16/06, A61J 17/00

(54) **A fluid guide for a soother**
Flüssigkeitsführung für einen Schnuller
Guide de fluide pour tétine

(30) Priority: 04.05.2010 DK 201070186
(43) Date of publication of application: 13.03.2013
(73) Proprietor: Region Nordjylland, Aalborg Sygehus, 9220 Aalborg Ø (DK)
(72) Inventor: BUCH, Søren, Grøndahl, DK-9000 Aalborg (DK); BJØRN, Signe, DK-9000 Aalborg (DK)
(74) Representative: Plougmann & Vingtoft A/S
(86) International application number: PCT/DK2011/050122
(87) International publication number: WO 2011/137905

(56) References cited:
- WO-A1-2006/125610
- WO-A1-2009/109005
- US-A1- 2009 173 349
- US-A1- 2010 000 525
- US-A1- 2010 147 298
- US-B1- 6 799 575

## Description

### FIELD OF THE INVENTION

The invention relates to a soother for guiding fluid to be inhaled by a child.

### BACKGROUND OF THE INVENTION

When children need supply of additional oxygen, e.g. after having been in anesthesia, oxygen is typically supplied via a catheter guided into the nostrils or by a mask placed above the child's nose and mouth. Such methods are accomplished by inconvenience and particularly infants seldom accept such breathing aid. In consequence, such methods may not be used with infants. The most common way of supplying additional oxygen to the child is by blowing oxygen towards the nose. However, by this method it Is uncertain if the oxygen directed sufficiently accurate to be efficiently inhaled. Furthermore, the child may turn it head away from the source of oxygen.

Accordingly, improved methods for breathing aid and supply of oxygen or other fluids to be inhaled by infants are needed.

WO2006125610 discloses a pacifier for sedation. The pacifier sprays oxygen loaded with sedative near the patient's nostrils through conducting tubes with movable bases connected to the source of the sedative gas. Such pacifier is used for sedating baby patients who need surgical operations, In lieu of the sedation mask used in all hospitals nowadays.

US 5375593 discloses a pacifier having a nipple, a mouth plate, a housing assembly and a pair of nasal cannula coupled to one another and extending out of the housing so that whenever the infant has the nipple in his or her mouth the nasal cannulae are automatically directed to each nostril of the infant. A provision is made for connection of the nasal cannulae to an external oxygen source. In one embodiment a bladder is interposed between the nasal cannulae and the external oxygen source to ensure that even distribution of oxygen from the external source is provided to each nostril. A hole in the bladder makes a tight seal around an oxygen line which terminates inside the bladder by way of an input port which is formed by the end of the oxygen line. Another embodiment has the nasal cannulae coupled to one another by a "T" or "Y"-connection and that "T" or "Y"-connection directly coupled to the external oxygen source.

WO 2006125610 relates to a pacifier for sedation. The pacifier sprays oxygen loaded with sedative near the patient's nostrils through conducting tubes with movable bases connected to the source of the sedative gas. Two conducting tubes are connected to a source of sedative gas and oxygen mixture and another end of the two tubes are connected to gas conducting tubes opposite to the nostrils of the baby to be sedated. Such pacifier is used for sedating baby patients who need surgical operations, in lieu of the sedation mask used in all hospitals nowadays.

US2005/0263157 discloses a breathing aid for an infant adapted for delivery of inhalable medication in a gentle flow to an area immediately adjacent the baby's nasal passageway. An oral member formed as a pacifier carries a hollow tube, the open top end of which extends just above the upper rim of the mouth guard of the pacifier. A swivel connector secured to the bottom of the tube allows easy adjustment of the position of the breathing aid in relation to the mouth guard and to a connecting hose, through which oxygen or other medication is delivered.

Whereas the breathing aid disclosed by US2005/0263157 may be seen as an improvement of breathing aids for infants, the inventor has appreciated that an improved soother with improved fluid delivery capabilities and improved construction is of benefit, and has in consequence devised the present invention.

### SUMMARY OF THE INVENTION

In general it would be advantageous to achieve an improvement of breathing aids and other fluid delivering means for use with children. It would also be desirable with a soother for aiding inhalation of fluids which has improved function, use, and manufacturing capabilities. In general, the invention preferably seeks to mitigate, alleviate or eliminate one or more of the above mentioned disadvantages singly or in any combination. In particular, it may be seen as an object of the present invention to provide a method that solves the above mentioned problems, or other problems, of the prior art.

To better address one or more of these concerns, in a first aspect of the invention a fluid guide for a soother is presented, where the soother comprises a mouth shield, where the fluid guide is adapted for guiding a fluid to be inhaled by a child, and where the fluid guide is engageable with a back side of the mouth shield, the fluid guide comprising first and second fluid outlets for delivering the fluid to be inhaled and first and second fluid inlets connectable with a fluid delivering source, where the first outlet and the first inlet are formed as a first conduit, the second outlet and the second inlet are formed as a second conduit, and where the first and second conduits are connected by a connector integrally formed with the inlets and the outlets.

The fluid guide may be fixedly engageable with the mouth shield so that the fluid guide is in a fixed and locked engagement with the mount shied when the fluid guide and the mouth shield are assembled. As an example, the engagement may be enabled via the connector or other structural part being integrally shaped with and comprised by the fluid guide, where the connector or other structural part is shaped to enable a locked engagement with the mouth shield.

The first and second conduits and the connector - i.e. the inlets, the outlets and the connector - are formed as a single structural part in a single type of material. Since the fluid guide may be formed as a single piece, the manufacturing of the fluid guide may be simplified in comparison with fluid guides consisting of several parts.

Furthermore, the single piece part, i.e. the integrally formed fluid guide, may be designed to be easily connected to other parts of a soother, such as the mouth shield of a soother. For example, the connector, which may be solid, may be used for connecting the fluid guide to receiving features of a part of the soother. In this way the fluid guide may be designed to be connected with ordinary soothers. I.e. an ordinary soother may be disassembled completely or partially and the fluid guide may be connected to a part of the soother. The fluid guide may also be connected to an extremity of a soother which is not separable. Thus, the fluid guide may have a connection means enabling the connection with other parts of a soother or extremities of a soother. Accordingly, a child's preferred soother may be used with the fluid guide implying that that child is more encouraged to take the soother. The connection between the first and second conduits may be rigid so that the first and second conduits are non-moveable relative to each other.

The single piece construction of the soother implies that the fluid guide may be made rigid so that when the fluid guide is mounted on the soother then the outlet of the fluid guide does not unintentionally displace from the optimal position below the nostrils. Thereby it may be avoided that the efficiency of the fluid supply aid is lowered and for example that dry oxygen is blown towards the eyes and causes drying of the eyes.

The fluid guide is applicable for guiding gases, such as oxygen, to the nostrils. However, the fluid guide may also be used for guiding liquid, such as water to the nostrils. The supply of liquid may be used to clean and remove vomit.

Since the fluid guide may comprise first and second fluid outlets, an outlet may be provided for guiding fluids to each nostril.

Since the fluid guide comprises first and second fluid inlets, individual fluid supplies or fluid hoses may be connected to inlets to supply the fluid to each outlet. Since standard fluid delivering hoses such as oxygen hoses normally has to two output hoses, it may be an advantage to have two fluid inlets so that standard fluid hoses can be connected to the fluid guide.

In an embodiment the first and second outlets are located within the extension of the mouth shield, i.e. the outlets do not protrude beyond the rim or the outer contour of the mouth shield. Thereby, it is more likely that the child will accept the soother with a fluid guide in place of the child's normal soother since the soother does not interfere with the child's nose.

In an embodiment the first and second conduits are formed from a plastic material having a stiffness enabling the locations of the first and second outlets to be fixed relative to the mouth shield when the fluid guide is engaged with the back side of the mouth shield. The first and second conduits may advantageously be manufactured in a relatively stiff material so that the outlets do not displace. The conduits may also advantageously be made stiff to ease the insertion of the fluid delivering hoses.

In an embodiment the first and second conduits forms separate first and second unconnected fluid passageways. Accordingly, fluid being provided to the first inlet is not able to mix with the fluid being provided to the second inlet. Thereby, the flow in a first fluid supply hose connectable with the first fluid inlet and the flow in a second fluid supply hose connectable with the second fluid inlet are not able to interfere with each other during the passage from the inlets to the outlets.

In an embodiment the openings of the first and second inlets and the first and second outlets have the same circular diameters. Since the cross-section of each of the first and second passageways of the first and second conduits is the same all the way from the inlets to the outlets the fluid guide may be manufactured in a particularly simple way. Furthermore, since the diameter does not change from the inlet to the outlet, the fluid supply hoses may be pushed almost all the way through the conduits up to the outlet to ensure that the fluid supply hoses do not slip out of the conduits.

In an embodiment the connector may be releasably connectable with a fastening means of the mouth shield for a fixed connection. The connector may advantageously be releasably connectable so that the fluid guide can be separated from the soother after use, so that the soother and/or the fluid guide can be reused. This may be particularly advantageous when the fluid guide is connected to an ordinary soother which is also used without the fluid guide.

In an embodiment each inlet comprises a cylindrical hole, i.e. first and second holes, configured to receive and hold a fluid delivering hose. The fluid delivering hose may advantageously be inserted into the inlet for eased connection of the hoses and so that the conduits are allowed to be located close to the mouth shield. Thus, each of the first and second cylindrical holes may protrude into each of the respective first and second conduits and may form through holes.

In an embodiment the distance between the first and second outlets are smaller than the distance between the first and second inlets. Accordingly, the outlets can be adapted to the distance between nostrils, whereas the inlets can be designed to obtain the most practical hose connection.

In an embodiment fluid hoses for supplying fluid to the first and second conduits are fixated to the first and second fluid inlets.

In an embodiment each of the first and second inlets and portions of each of the first and second conduits connected to the inlets has a first diameter, and each of the first and second outlets and portions of each of the first and second conduits connected to the outlets has a second diameter being smaller than the first diameter.

In an embodiment according one of the embodiments, the first diameter is substantially equal to the outer diameter of the fluid hoses and the second diameter is substantially equal to the diameter of the lumen of the fluid hoses.

A second aspect of the invention relates to a soother for guiding a fluid to be inhaled by a child, the soother comprising
- a mouth shield,
- a nipple for the mouth of the child, where the nipple extends from a front side of the mouth shield,
- a fluid guide according to the first aspect.

It is understood that the fluid guide may be sold separately from soothers since the fluid guide may be connectable with ordinary soothers which may be separable into soother parts or may not be separable. Alternatively, a soother with a connectable fluid guide may be manufactured and sold as an item.

In an embodiment the soother comprises a locking part releasably connectable with the mouth shield and the connector for fixing the fluid guide relative to the mouth shield. A locking part may advantageously be provided for connecting the fluid guide to the soother. The locking part may be part of the soother or the locking part may be a part specially designed to connect the fluid guide to an ordinary soother.

A third aspect of the invention relates to a a method for manufacturing a soother suited for guiding a fluid to be inhaled by a child, the method comprises
- providing a soother,
- connecting a fluid guide to the soother, the fluid guide comprises first and second fluid outlets for delivering the fluid to be inhaled and first and second fluid inlets connectable with a fluid delivering source, where
   each of the first outlet and the first inlet constitutes openings of a first conduit, each of the second outlet and the second inlet constitutes openings of a second conduit, and where the first and second conduits are connected by a connector connectable with the soother and integrally formed with the first and second conduits.

The fluid guide may be designed to be connected to different commercially available soothers, i.e. different fluid guides may be manufactured for different soothers. Alternatively, a soother specifically designed for used with a fluid guide may be manufactured.

In an embodiment the fluid guide is for a soother comprising a mouth shield, where the fluid guide is adapted for guiding a fluid to be inhaled by a child, and where the fluid guide is engageable with a back side of the mouth shield, the fluid guide comprises a fluid outlet for delivering the fluid to be inhaled and a fluid inlet connectable with a fluid delivering source, where the inlet and the outlet are integrally formed as a single piece.

In general the various aspects of the invention may be combined and coupled in any way possible within the scope of the invention. These and other aspects, features and/or advantages of the invention will be apparent from and elucidated with reference to the embodiments described hereinafter.

In summary the invention relates to a soother for breathing aid. The soother is provided with a fluid guide comprising one or more conduits for supplying e.g. oxygen to the nostrils of an infant. The fluid guide is manufactured as a single piece to ensure a simple design. The soother can be assembled from three or four parts which make the manufacturing of the soother simple. The soother may be designed so that parts of the soother are releasably connectable. Thereby, the soother may be disassembled after use so that efficient cleaning is possible. Accordingly, the cleaned parts can be assembled possibly in combination with new parts and other part types. The design of the fluid guide ensures convenient supply of e.g. oxygen to the nostrils and convenient connection of oxygen hoses.

### BRIEF DESCRIPTION OF THE DRAWINGS

Embodiments of the invention will be described, by way of example only, with reference to the drawings, in which
Fig. 1 shows an exploded view of a first soother with a fluid guide adaptor,
Fig. 2 shows perspective views, side, top, bottom, front and back views of a part of the first soother,
Fig. 3 shows perspective views, side, front, back and top views of the assembled first soother,
Fig. 4 shows a perspective view of a partly assembled first soother,
Fig. 5 shows an exploded view of a second soother with a different fluid guide adaptor,
Fig. 6 shows perspective views, side, front, back and top views of the assembled second soother,
Fig. 7 shows a perspective view of the second soother when fluid supply hoses are inserted,
Fig. 8A shows a cross sectional view of the fluid guide, and
Fig. 8B shows a cross sectional view of the fluid guide with fluid supplying means such as flexible hoses fixated to the inlets.

### DETAILED DESCRIPTION OF EMBODIMENTS

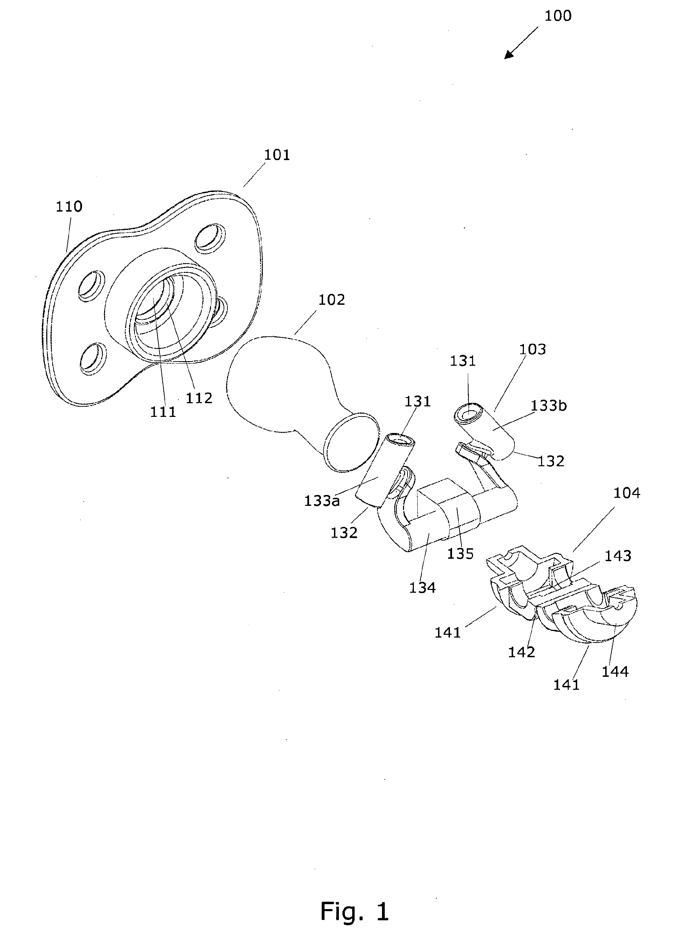

Fig. 1 shows parts, in an exploded view, of a soother 100 for guiding fluid such as gas containing oxygen to the nostrils of a child so that the child can inhale the gas. The soother comprises a mouth shield 101, a nipple 102 to be sucked on when placed in the mouth of the child, a fluid guide 103 for guiding the fluid and possible a locking part 104 for fixing the fluid guide 103 relative to the mouth shield 101.

The nipple 102 may be made of flexible material such as latex or silicone, whereas the remaining parts may be made of rigid or semi-rigid material such as ABS plastic, nylon or similar materials.

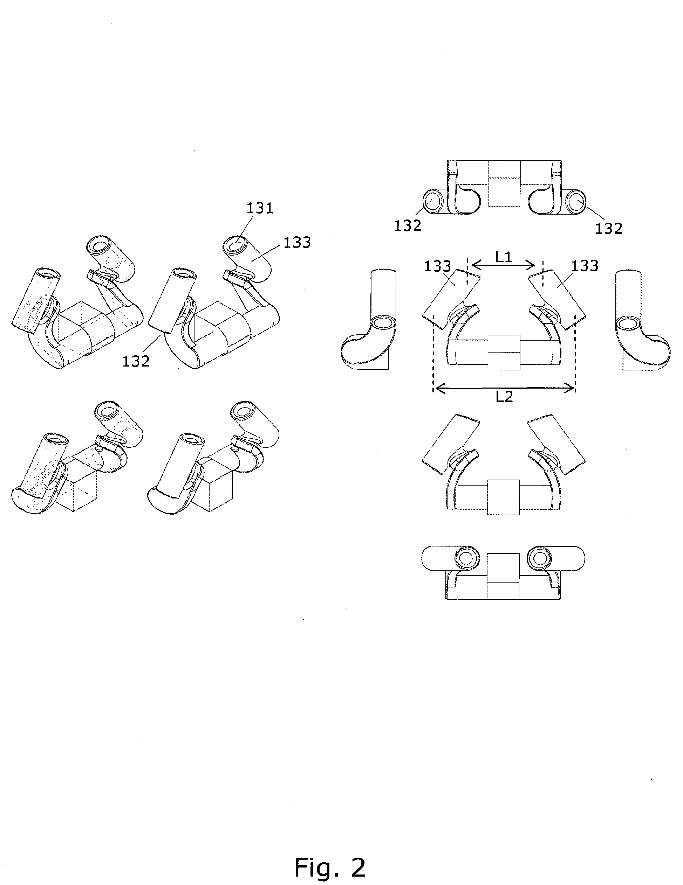

In the following reference is made to Figs. 1-4 for the description of the soother. In the figures identical reference numbers refer to the same or equivalent elements. Accordingly, where an element has already been described in relation to one figure, the same elements may not be described in relation to other figures. Where it is clear that figures show the same or corresponding elements reference number may have been omitted.

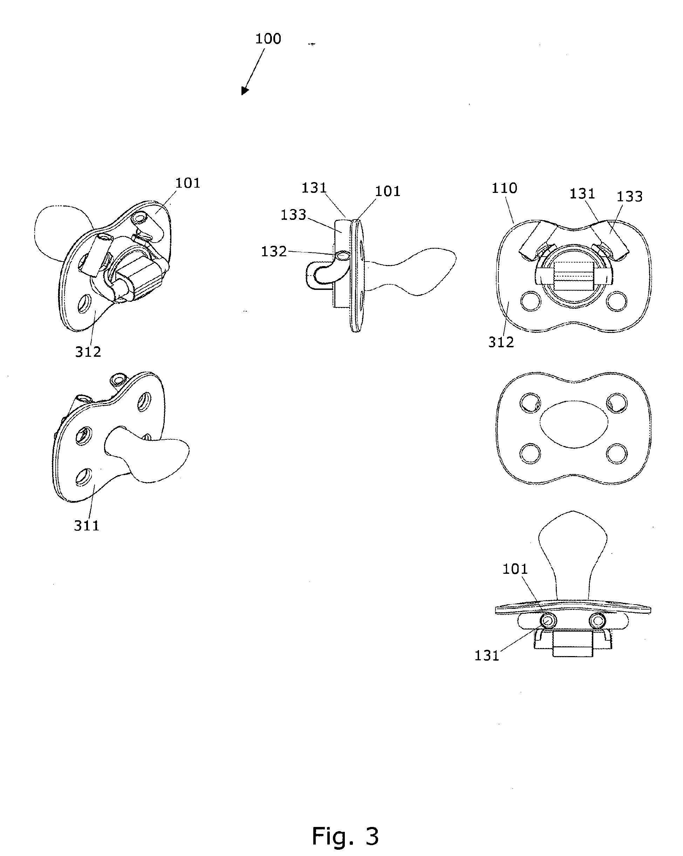

The mouth shield 101 may have different shapes and outer contours 110 as known from commercially available soothers. The mouth shield 101 has a through hole 111 with a flange 112 for receiving and holding the nipple 102 so that the nipple is secured from being drawn out. When assembled, the nipple extends from the front side 311 as shown in Fig. 3.

The fluid guide 103 comprises first and second fluid outlets 131 and first and second inlets 132. The first inlet and first outlet constitutes the openings of a first straight cylindrically shaped conduit 133. Similarly, the second inlet and the second outlet constitute the openings of a second straight cylindrically shaped conduit 133. Whereas the conduits 133 are illustrated as straight cylindrically shaped tubes, they may be curved, have shapes different than cylindrical and they may have different areas of the openings for the inlet 131 and the outlet 132.

The first and second conduits 133 are connected by a connector 134. The connector 134 may be solid. Accordingly, the inlets 131, outlets 132 and conduits 133 are integrally formed from a single piece, and preferably from a single piece of material.

The cylindrical hole of each inlet 132 can receive and hold a hose for delivering the fluid, e.g. oxygen. Accordingly, the inner diameter of the hole of the inlet 132 is preferably slightly smaller than the outer diameter of the delivering hose so that the hose is securely fixed to the inlet by pressure.

The soother 100 may comprise a locking part 104 for fixing the connector 134 to the mouth shield 102. The locking part 104 is formed as two identical parts 141 connected via a hinge part 142, where the identical parts and the hinge is integrally formed as a single piece of a single material. The identical parts 141 comprise indentations 143 for receiving corresponding mating protrusions 135 of the fluid guide 104.

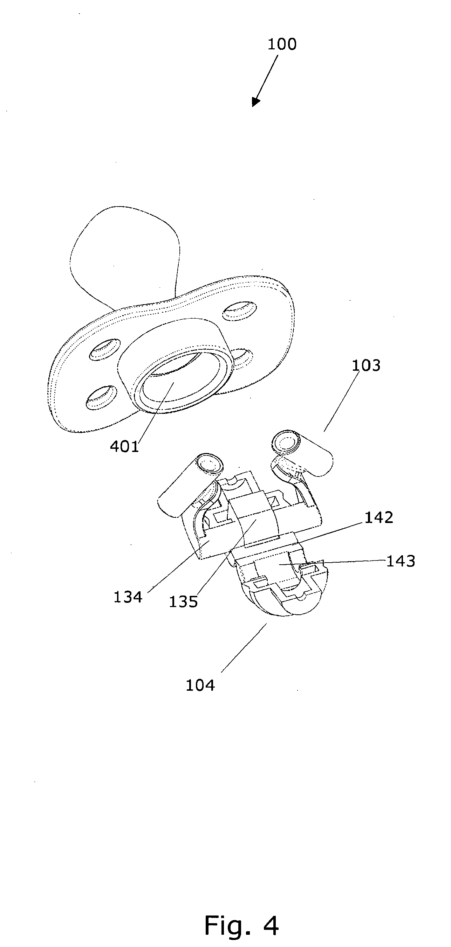

Fig. 4 shows how the protrusion 135 of the fluid guide 104 is accommodated by the mating indentations 143 of the locking part 104. After the locking part 104 is closed around the connector 134 using the hinge 142, the assembled part can be inserted in the hole 401 of the mouth shield 102. Accordingly, the fluid guide 103 is engageable with the back side 312 of the mouth shield by means of the locking part 104 which is able to fix the connector 134 to the mouth shield 101.

It is appreciated that the locking part 104 may have different shapes depending on the shape of the soother or the mouth shield.

The closed locking part 104 forms a cylindrical insert 144 which can be press fitted into the cylindrical hole 111 of the mouth shield. Since the insert 144 is rotation symmetric, the same locking part can be rotated by any angle before it is inserted into the mouth shield. This may be useful for connecting other types of fluid guides to the shield 102, e.g. a fluid guide with a single conduit 133 which extends in the same direction as the connector 134.

The distance L1 between the first and second outlets 131 may be smaller than the distance L2 between the first and second inlets 132. Thus, the first and second conduits 131 may be angled so that the distance L1 between the outlets 131 are optimized to match the distance between typical nostrils, and the distance between the inlets 132 may be optimized for eased connection of hoses. Additionally, the conduit 133 may be angled or inclined towards the mouth shield, so that when the soother is in use the conduit or conduits 133 points in an inward direction towards the face of the child, so that the direction of the fluid stream is adapted to the direction of the nostrils to optimize the efficiency of the uptake or inhalation of fluid.

All parts of the soother 100 including any of the mouth shield 101, the nipple 102, the fluid guide 103 and the locking part 104 may be releasably connectable so that the parts can be disconnected after use. For example, it may be desirable to reuse some or all of the parts, for example the mouth shield 101, the fluid guide 103, and the locking part 104 could be reused after cleaning, whereas the nipple 102 is not reused. The releasable parts may also enable assembly of soothers 100 from different types of parts. For example, nipples 102 of different sizes may be used with the same type of mouth shields 101. Also, different types of fluid guides 103 may be available, e.g. fluid guides with different distances between outlets 131 may be available to enable selection of a fluid guide which matches the nostrils of children of different age. In this way the soother 100 can be optimized for use with different patients to achieve efficient fluid uptake.

Fig. 3 shows the assembled soother 100 in perspective views and in side, front, back and top views. The side view and the back view (of the back side 312) show that the outlet 131 is in line with the border 110 of the mouth shield. It may be an advantage that the outlet 131 is within the border 110 of the mouth shield 101 or is formed to be in line with a part of the border 110 of the mouth shield, i.e. so that the conduit 133 and the outlet 131 does not protrude with the effect that the outlets 131 cannot easily interfere with the nose or nostrils and, thereby, annoy the child. Furthermore, since the hose can be inserted into the hole of the inlet 132, the conduit 133 can be located close to or in contact with the face of the front side 312 of the mouth shield. Thereby, it can be achieved that the outlets 131 can be located just below the nostrils, i.e. close to the skin, to optimize the uptake of the fluid by the child.

Whereas it may be advantageous that the outlet 131 does not protrude outside the rim 110 of the mouth shield for some applications, there may exist other applications where the conduits 133 and outlet 131 should extend into the one or both nostrils to ensure a better inhalation of the fluid or the force the fluid into the nostrils. Thus, in an embodiment the conduits 133 extend beyond the rime 110.

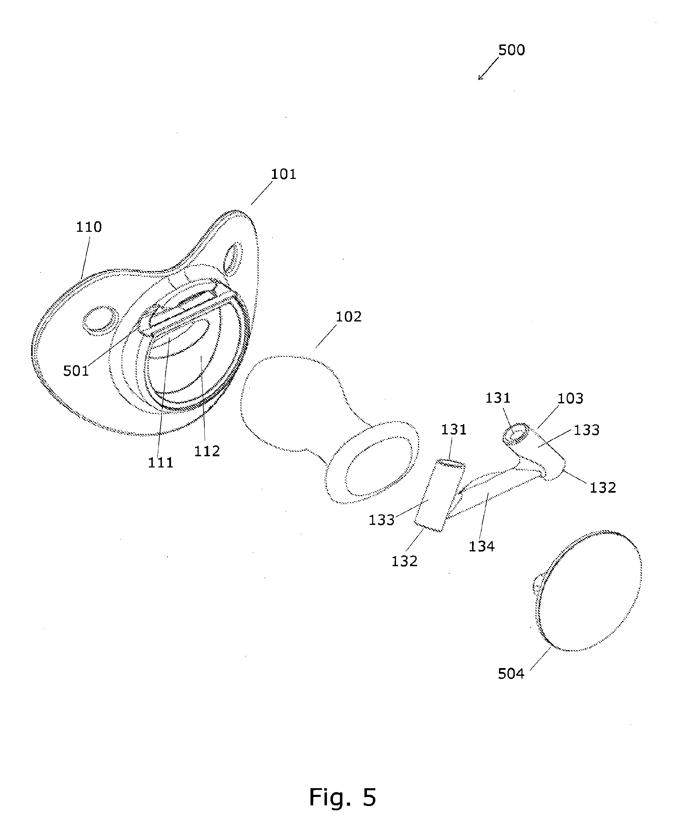

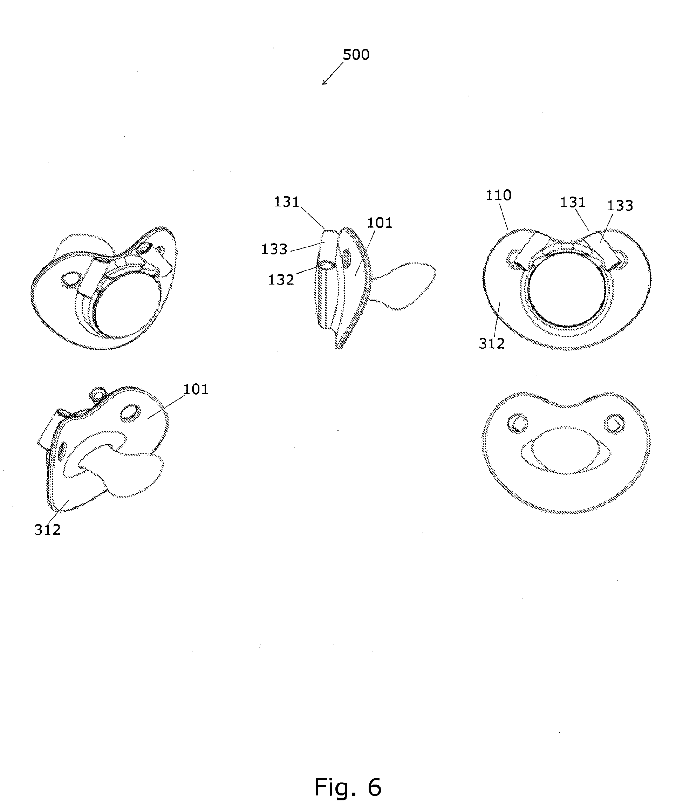

Figs. 5-7 show a soother 500 which is functionally equivalent to the soother 100. The soother 500 generally have the same features as the soother 100 and, therefore, features of soother 500 which are equivalent with features of soother 100 bear the same reference numbers. Accordingly, where features of soother 100 and soother 500 are equivalent the description of features of soother 100 applies equally to soother 500.

The mouth shield 101 of the soother 500 differs from the mouth shield 101 of the soother 100 by having a fastening means 501, such as a recess or notch, for fixation of the fluid guide 103 of the soother 500. Accordingly, the fluid guide 103 can be fixed directly to mouth shield 101 without use of a locking part 104. Therefore, the fluid guide 103 of the soother 500 need not have protrusions 135 for engagement with the locking part, but may be directly engageable with the back side 312 of the mouth shield 103.

The fluid guide 103 of the soother 500 has a first conduit 133 with a first inlet and a second outlet which is connected to a second conduit 133 via connector 134. Accordingly, the inlets 131 and outlets 132 are integrally formed as a single piece, preferably of a single material.

The soother 500 also comprises a locking part 504 which is equivalent with the locking part 104 of the soother 100 except that it does not have indentations 143 for receiving protrusions 135 of the fluid guide 103. The locking part 504 can be pressed onto mouth shield 101 to establish additional fixation of the fluid guide 103.

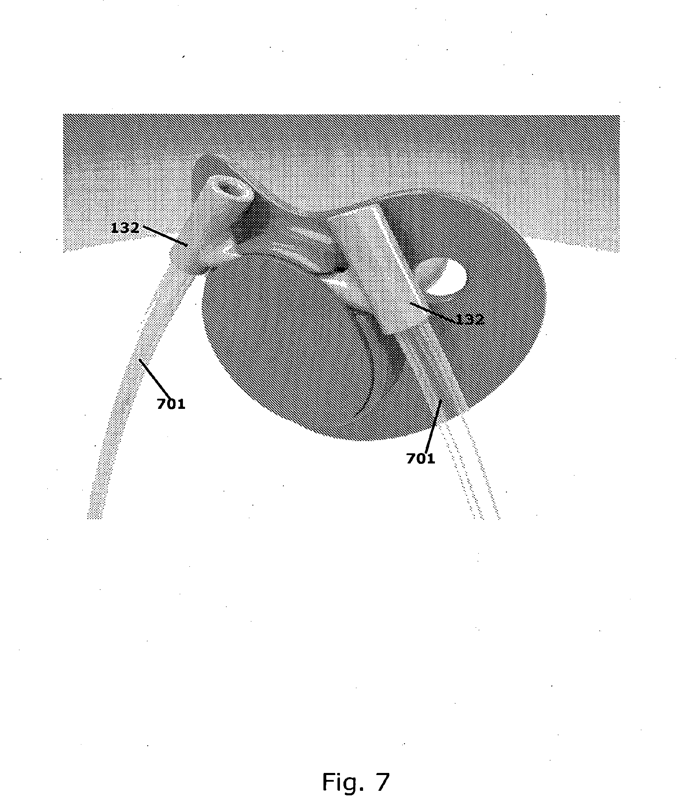

Fig. 7 shows the assembled soother 500 where hose or tubes 701 have been inserted into the inlets 132 of the fluid guide.

Due to the releasable parts of the soother the fluid guide 103 may designed to fit ordinary soothers, i.e. soothers without fluid guide capabilities. For example the connector 134 and the protrusion 135 of Figs. 1-4 may be shaped to fit a commercially available soother which may be disassembled similarly to the soother 100. Similarly, the connector 134 of the soother of Figs. 5-7 may have been shaped to fit the recess 501 of an existing soother.

It is appreciated that the fluid guide 103 may also be used with ordinary soothers which are not readily separable. In this case the fluid guide 103 may be provided with connection means or locking means which enables the fluid guide 103 to be connected to a soother. For example, the fluid guide 103 may be provided with integrally formed clips which can be clipped onto the rim 110 of the mouth shield, or the fluid guide 103 may be provided with a sticky portion enabling the fluid guide to be attached to any part of the ordinary soother, e.g. a surface of the mouth shield.

Accordingly, an aspect the invention relates to the fluid guide 103. In this aspect, the fluid guide may advantageously be designed as a single piece of a single material so that it can easily be used with separable ordinary soothers. Thus, ordinary soothers may be utilized with fluid guides 103 so that the ordinary soother can be used e.g. as a breathing aid soother.

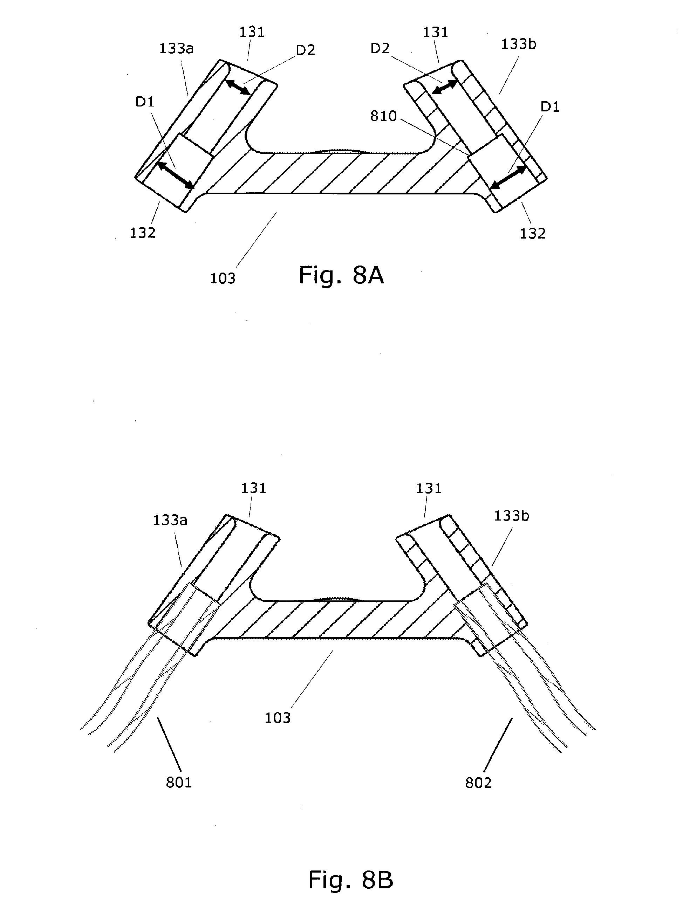

Fig. 8A shows a cross sectional view of an embodiment of a fluid guide 103 applicable to any of the fluid guides of the invention. According to the embodiment of Fig. 8A, each of the first and second inlets 132 and first portions of each of the first and second conduits 133a, 133b connected to the inlets has a first diameter D1, and each of the first and second outlets 131 and second portions of each of the first and second conduits 133a, 133b connected to the outlets has a second diameter D2 being smaller than the first diameter D1. Thus, the conduits 133 may have cylindrical holes with a first diameter D1 extending from the inlets towards the outlets, and cylindrical holes with a second diameter D2 extending from the outlets towards the inlets. Advantageously, the first and second portions of the conduits may adjoin, so that the cylindrical holes with a first diameter D1 extend from the inlets to the cylindrical holes with the second diameter so that an end of one of the cylindrical holes with the smaller second diameter D2 forms a rest 810 against which a fluid hose can be pressed against.

It is understood that the outlets and inlets may have rounded edges near the ends of the conduits. However, the first and second diameters are understood does not include dimensions of such rounded edges but refer to the cylindrical diameters of the conduits.

In an advantageous embodiment the first diameter D1 is substantially equal to the outer diameter of the fluid hoses and the second diameter D2 is substantially equal to the diameter of the lumen of the fluid hoses. Thereby, the fluid passageway extending from the fluid hoses 701 to the conduits is substantially continuous so that fluid expelled from the fluid hoses 701 into one of the conduits 133 does not encounter any edges or changes in the diameter of the fluid passageway. Since discontinuities, edges or changes of the diameter of the fluid passageway may cause whistle noise, this embodiment may be particularly advantageous for avoiding annoying noise due to the air flow.

Fig. 8B shows an embodiment where fluid hoses 701, 801, 802 for supplying fluid to the first and second conduits 133a, 133b are fixated to the first and second fluid inlets (132). The fluid hoses may be fixated during manufacturing of the fluid guide by gluing the fluid hoses to the inlets, e.g. by applying UV cureable adhesive to the outer circumference of the fluid hoses and applying UV light to cure the adhesive. According to the embodiment where the fluid hoses are fixated, the first portion of the conduits 133 may advantageously have a diameter D1 and the second portion of the conduits may have a smaller diameter D2 so as to form a rest 810 against which a fluid hose can be pressed against for eased manufacturing of the fixated fluid hoses 801, 802.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. Any reference signs in the claims should not be construed as limiting the scope.

## Claims

1. A fluid guide (103) for a soother (100), the soother comprising a mouth shield (101), where the fluid guide is adapted for guiding a fluid to be inhaled by a child, and where the fluid guide is engageable with a back side (312) of the mouth shield, the fluid guide comprising first and second fluid outlets (131) for delivering the fluid to be inhaled and first and second fluid inlets (132) connectable with a fluid delivering source, where the first outlet and the first inlet are formed as a first conduit (133a), the second outlet and the second inlet are formed as a second conduit (133b), and where the first and second conduits are connected by a connector (134), **characterized in that** the connector is integrally formed as a single piece with the inlets and the outlets.

2. A fluid guide according to claim 1, where the first and second outlets (131) are located within the extension of the mouth shield (101).

3. A fluid guide according to claim 1, where the first and second conduits (133a,133b) are formed from a plastic material having a stiffness enabling the locations of the first and second outlets (131) to be fixed relative to the mouth shield when the fluid guide is engaged with the back side (312) of the mouth shield.

4. A fluid guide according to claim 1, where the first and second conduits (133a, 133b) forms separate first and second unconnected fluid passageways.

5. A fluid guide according to claim 1, where openings of the first and second inlets and the first and second outlets have the same circular diameters.

6. A fluid guide according to claim 1, where the connector (134) is releasably connectable with a fastening means (501) of the mouth shield for a fixed connection.

7. A fluid guide according to any of the preceding claims, where each inlet comprises a cylindrical hole configured to receive and hold a fluid delivering hose.

8. A fluid guide according to claim 7, where each of the first and second cylindrical holes protrudes into the each of the respective first and second conduits.

9. A fluid guide according to any of the preceding claims, where the distance between the first and second outlets are smaller than the distance between the first and second inlets.

10. A fluid guide according to any of the preceding claims, where fluid hoses (701, 801, 802) for supplying fluid to the first and second conduits are fixated to the first and second fluid inlets (132).

11. A fluid guide according to any of the preceding claims, where each of the first and second inlets and portions of each of the first and second conduits connected to the inlets has a first diameter (D1), and each of the first and second outlets and portions of each of the first and second conduits connected to the outlets has a second diameter (D2) being smaller than the first diameter.

12. A fluid guide according to claim 11 when dependent on claim 10, where the first diameter is substantially equal to the outer diameter of the fluid hoses and where the second diameter is substantially equal to the diameter of the lumen of the fluid hoses.

13. A soother (100) for guiding a fluid to be inhaled by a child, the soother comprising
- a mouth shield (101),
- a nipple (102) for the mouth of the child, where the nipple extends from a front side of the mouth shield,
- a fluid guide (103) according to claim 1.

14. A soother according to claim 13, comprising a locking part (504) releasably connectable with the mouth shield (101) and the connector (134) for fixing the fluid guide relative to the mouth shield.

15. A method for manufacturing a soother suited for guiding a fluid to be inhaled by a child, the method comprises
- providing a soother,
- connecting a fluid guide (103) to the soother, the fluid guide comprises first and second fluid outlets (131) for delivering the fluid to be inhaled and first and second fluid inlets (132) connectable with a fluid delivering source, where each of the first outlet and the first inlet constitutes openings of a first conduit (133a), each of the second outlet and the second inlet constitutes openings of a second conduit (133b), and where the first and second conduits are connected by a connector (134) connectable with the soother **characterized in that** the connector is integrally formed as a single piece with the first and second conduits.

## Patentansprüche

1. Flüssigkeitsführung (103) für einen Schnuller (100), wobei der Schnuller einen Mundschild (101) umfasst, wobei die Flüssigkeitsführung dazu beschaffen ist, eine von einem Kind zu inhalierende Flüssigkeit zu führen und wobei die Flüssigkeitsführung in einer Rückseite (312) des Mundschilds eingreifbar ist, wobei die Flüssigkeitsführung einen ersten und einen zweiten Flüssigkeitsauslass (131) zur Abgabe der zu inhalierenden Flüssigkeit und einen ersten und einen zweiten Flüssigkeitseinlass (132) umfasst, der mit einer Flüssigkeitsabgabequelle verbindbar ist, wobei der erste Auslass und der erste Einlass als ein erster Kanal (133a) ausgebildet sind, wobei der zweite Auslass und der zweite Einlass als ein zweiter Kanal (133b) ausgebildet sind und wobei der erste und der zweite Kanal durch ein Verbindungsstück (134) verbunden sind, **dadurch gekennzeichnet, dass** das Verbindungsstück einstückig als ein Teil mit den Einlässen und Auslässen ausgebildet ist.

2. Flüssigkeitsführung nach Anspruch 1, wobei der erste und der zweite Auslass (131) innerhalb der Ausdehnung des Mundschilds (101) angeordnet sind.

3. Flüssigkeitsführung nach Anspruch 1, wobei der erste und der zweite Kanal (133a, 133b) aus einem Kunststoffmaterial mit einer Steifigkeit ausgebildet sind, die die in Bezug auf den Mundschild unveränderliche Position des ersten und des zweiten Auslasses (131) beim Eingriff der Flüssigkeitsführung in die Rückseite (312) des Mundschilds ermöglicht.

4. Flüssigkeitsführung nach Anspruch 1, wobei der erste und der zweite Kanal (133a, 133b) getrennte erste und zweite nicht verbundene Flüssigkeitswege bilden.

5. Flüssigkeitsführung nach Anspruch 1, wobei die Öffnung des ersten und des zweiten Einlasses sowie des ersten und des zweiten Auslasses einen kreisförmigen Durchmesser aufweist.

6. Flüssigkeitsführung nach Anspruch 1, wobei das Verbindungsstück (134) zur Bereitstellung einer festen Verbindung lösbar mit einem Befestigungsmittel (501) des Mundschilds verbindbar ist.

7. Flüssigkeitsführung nach einem der vorhergehenden Ansprüche, wobei jeder Einlass eine zylindrische Öffnung umfasst, die zum Aufnehmen und Festhalten eines flüssigkeitsabgebenden Schlauchs konfiguriert ist.

8. Flüssigkeitsführung nach Anspruch 7, wobei jede der ersten und der zweiten zylindrischen Öffnung in jeden des jeweiligen ersten und zweiten Kanals ragt.

9. Flüssigkeitsführung nach einem der vorhergehenden Ansprüche, wobei der Abstand zwischen dem ersten und dem zweiten Auslass geringer ist als der Abstand zwischen dem ersten und dem zweiten Einlass.

10. Flüssigkeitsführung nach einem der vorhergehenden Ansprüche, wobei Flüssigkeitsschläuche (701, 801, 802) zur Förderung von Flüssigkeit zum ersten und zweiten Auslass an dem ersten und dem zweiten Flüssigkeitseinlass (132) befestigt sind.

11. Flüssigkeitsführung nach einem der vorhergehenden Ansprüche, wobei jeder des ersten und des zweiten Einlasses und Abschnitte jedes des ersten und des zweiten mit den Einlässen verbundenen Kanals einen ersten Durchmesser (D1) aufweist und jeder des ersten und des zweiten Auslasses und Abschnitte jedes des ersten und des zweiten mit den Auslässen verbundenen Kanals einen zweiten Durchmesser (D2) aufweist, der geringer ist als der erste Durchmesser.

12. Flüssigkeitsführung nach Anspruch 11, wenn sich dieser auf Anspruch 10 bezieht, wobei der erste Durchmesser im Wesentlichen gleich dem Außendurchmesser der Flüssigkeitsschläuche ist und wobei der zweite Durchmesser im Wesentlichen gleich dem Durchmesser des Lumens der Flüssigkeitsschläuche ist.

13. Schnuller (100) zum Führen einer von einem Kind zu inhalierenden Flüssigkeit, wobei der Schnuller umfasst
- einen Mundschild (101),
- ein Mundteil (102) für den Mund des Kindes, wobei sich das Mundteil von einer Vorderseite des Mundschilds erstreckt,
- eine Flüssigkeitsführung (103) nach Anspruch 1.

14. Schnuller nach Anspruch 13, umfassend einen Verschlussteil (504), der zum Fixieren der Flüssigkeitsführung in Bezug auf den Mundschild lösbar mit dem Mundschild (101) und dem Verbindungsstück (134) verbindbar ist.

15. Verfahren zur Herstellung eines Schnullers, der zum Führen einer von einem Kind zu inhalierenden Flüssigkeit geeignet ist, wobei das Verfahren umfasst
- Bereitstellen eines Schnullers,
- Verbinden einer Flüssigkeitsführung (103) mit dem Schnuller, wobei die Flüssigkeitsführung einen ersten und einen zweiten Flüssigkeitsauslass (131) zur Abgabe der zu inhalierenden Flüssigkeit und einen ersten und einen zweiten Flüssigkeitseinlass (132) umfasst, der mit einer Flüssigkeitsabgabequelle verbindbar ist, wobei
jeder des ersten Auslasses und des ersten Einlasses einen ersten Kanal (133a) darstellt, jeder des zweiten Auslasses und des zweiten Einlasses einen zweiten Kanal (133b) darstellt und wobei der erste und der zweite Kanal durch ein mit dem Schnuller verbindbares Verbindungsstück (134) verbunden sind, **dadurch gekennzeichnet, dass** das Verbindungsstück einstückig als ein Teil mit dem ersten und dem zweiten Kanal ausgebildet ist.

## Revendications

1. Guide-fluide (103) pour une sucette (100), la sucette comprenant un bouclier (101), où le guide-fluide est adapté pour guider un fluide devant être inhalé par un enfant, et où le guide-fluide peut être engagé dans une face arrière (312) du bouclier, le guide-fluide comprenant une première et une deuxième sortie (131) de fluide pour amener le fluide devant être inhalé et une première et une deuxième entrée (132) de fluide raccordables à une source d'amenée de fluide, où la première sortie et la première entrée forment un premier conduit (133a), la deuxième sortie et la deuxième entrée forment un deuxième conduit (133b), et où le premier et le deuxième conduit sont raccordés par un raccord (134), **caractérisé en ce que** le raccord forme avec les entrées et les sorties une seule pièce intégrée.

2. Guide-fluide selon la revendication 1, où la première et la deuxième sortie (131) sont situées dans l'extension du bouclier (101).

3. Guide-fluide selon la revendication 1, où le premier et le deuxième conduit (133a, 133b) sont formés à partir d'une matière plastique dont la rigidité permet que l'emplacement de la première et de la deuxième sortie (131) soit fixe par rapport au bouclier quand le guide-fluide est engagé dans la face arrière (312) du bouclier.

4. Guide-fluide selon la revendication 1, où le premier et le deuxième conduit (133a, 133b) forment un premier et un deuxième passage de fluide distincts qui ne sont pas raccordés.

5. Guide-fluide selon la revendication 1, où les ouvertures de la première et de la deuxième entrée et de la première et de la deuxième sortie possèdent le même diamètre circulaire.

6. Guide-fluide selon la revendication 1, où le raccord (134) peut être raccordé de manière amovible à un moyen de fixation (501) du bouclier pour un raccordement fixe.

7. Guide-fluide selon l'une quelconque des revendications précédentes, où chaque entrée comprend un trou cylindrique configuré pour recevoir et maintenir un tuyau d'amenée de fluide.

8. Guide-fluide selon la revendication 7, où chacun des premier et deuxième trou cylindrique fait saillie dans chacun des premier et deuxième conduit correspondant.

9. Guide-fluide selon l'une quelconque des revendications précédentes, où la distance entre la première et la deuxième sortie est inférieure à la distance entre la première et la deuxième entrée.

10. Guide-fluide selon l'une quelconque des revendications précédentes, où les tuyaux de fluide (701, 801, 802) destinés à amener le fluide vers le premier et le deuxième conduit sont fixés sur la première et la deuxième entrée (132) de fluide.

11. Guide-fluide selon l'une quelconque des revendications précédentes, où chacune des première et deuxième entrée et des portions de chacun des premier et deuxième conduit raccordées aux entrées possède un premier diamètre (D1), et chacune des première et deuxième sortie et des portions de chacun des premier et deuxième conduit raccordées aux sorties possède un deuxième diamètre (D2) qui est inférieur au premier diamètre.

12. Guide-fluide selon la revendication 11 lorsqu'elle dépend de la revendication 10, où le premier diamètre est essentiellement égal au diamètre extérieur des tuyaux de fluide et où le deuxième diamètre est essentiellement égal au diamètre de la lumière des tuyaux de fluide.

13. Sucette (100) pour guider un fluide devant être inhalé par un enfant, la sucette comprenant
- un bouclier (101),
- une tétine (102) pour la bouche de l'enfant, où la tétine s'étend depuis une face avant du bouclier,
- un guide-fluide (103) selon la revendication 1.

14. Sucette selon la revendication 13, comprenant une pièce de blocage (504) pouvant être raccordée de manière amovible au bouclier (101) et au raccord (134) pour fixer le guide-fluide par rapport au bouclier.

15. Procédé de fabrication d'une sucette adaptée pour guider un fluide devant être inhalé par un enfant, le procédé comprenant
- l'apport d'une sucette,
- le raccordement d'un guide-fluide (103) à la sucette, le guide-fluide comprenant une première et une deuxième sortie (131) de fluide pour amener le flux devant être inhalé et une première et une deuxième entrée (132) de fluide pouvant être raccordées à une source d'amenée de fluide, où la première sortie et la première entrée constituent chacune des ouvertures d'un premier conduit (133a), la deuxième sortie et la deuxième entrée constituent chacune des ouvertures d'un deuxième conduit (133b), et où le premier et le deuxième conduit sont raccordés par un raccord (134) pouvant être raccordé à la sucette **caractérisé en ce que** le raccord forme avec le premier et le deuxième conduit une seule pièce intégrée.
